# EUROPEAN PATENT APPLICATION

(11) **EP 3 366 281 A1**
(43) Date of publication of application: **29.08.2018**
(21) Application number: 18158572.0
(22) Date of filing: 26.02.2018
(51) Int. Cl.: A61K 9/20, A61K 31/55

(54) **SOLID ORAL PHARMACEUTICAL COMPOSITIONS OF IVABRADINE**

(30) Priority: 28.02.2017 TR 201703066
(71) Applicant: Sanovel Ilac Sanayi ve Ticaret A.S., 34460 Istanbul (TR)
(72) Inventor: TÜRKYILMAZ, Ali, 34460 Istanbul (TR); PALANTÖKEN, Arzu, 34460 Istanbul (TR); GÜNER, Dicle, 34460 Istanbul (TR)
(74) Representative: Sevinç, Erkan

(57) **Abstract**

The present invention relates to solid oral pharmaceutical compositions, comprising ivabradine hydrochloride or ivabradine oxalate or pharmaceutically acceptable polymorphs thereof.

## Description

### Field of the invention

The present invention relates to solid oral pharmaceutical compositions, comprising ivabradine hydrochloride or ivabradine oxalate or pharmaceutically acceptable polymorphs thereof.

### Background of the invention

Ivabradine, (marketed under the trade name Corlanor) is a medication used for the symptomatic management of stable heart related chest pain and heart failure not fully managed by beta blockers.

The chemical name of ivabradine is 3-{3-[{[(7S)-3,4-dimethoxybicyclo[4.2.0]octa-1,3,5-trien-7-yl]methyl}(methyl)amino]propyl}-7,8-dimethoxy-1,3,4,5-tetrahydro-2H-3-benzazepin-2-one and its chemical structure is shown in the Formula 1.

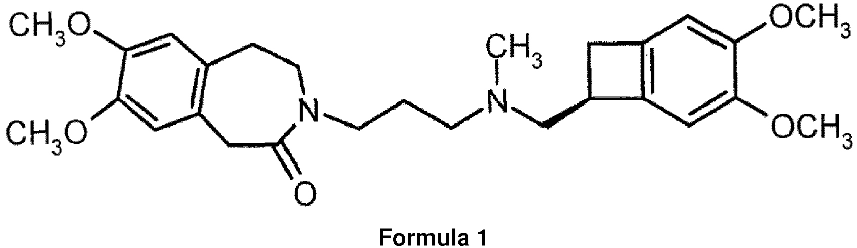

Ivabradine is a heart rate lowering agent. It acts by selective and specific inhibition of the cardiac pacemaker If current, an important ionic current that usually controls spontaneous diastolic depolarisation in the sinus node and thereby regulates heart rate.

Ivabradine molecule is disclosed in EP0534859. Ivabradine hydrochloride exhibits polymorphism. WO2006092493 discloses polymorph β of ivabradine hydrochloride, its process of preparation and compositions comprising this polymorph. Polymorph β is the most stable form and is present in the marketed product. Other polymorphic forms of ivabradine hydrochloride are disclosed in WO2005110993, WO2006092491, WO2006092492, WO2006092494, WO2007042656, WO2007042657 and WO2013064307. It's known from the prior art, ivabradine hydrochloride crystallizes easily and and polymorphic transitions of ivabradine hydrochloride take place rather easily, especially in drug products.

Amorphous ivabradine hydrochloride and methods for its preparation are disclosed in WO2008146308, CN101597261 and CN101463008.

### Detailed description of the Invention

The main object of the present invention is to provide solid oral pharmaceutical compositions comprising ivabradine or a pharmaceutically acceptable salt thereof or pharmaceutically acceptable polymorphs thereof, having a content uniformity with high stability and bioavailability, wherein the pharmaceutically acceptable salt is ivabradine hydrochloride or ivabradine oxalate.

It's known that ivabradine hydrochloride exhibits polymorphic transitions easily. Different polymorphs can lead to differences in solubility, dissolution profiles, and therefore bioavailability and stability of the product. Stable products should not undergo polymorphic transformation during their production and shelf life.

In one embodiment, the solid oral composition comprises ivabradine hydrochloride or ivabradine oxalate or pharmaceutically acceptable polymorphs thereof in an amount of 0.01% to 20.00% by weight of the total formulation, preferably 0.2% to 20.00.

According to this embodiment, the composition is in the form of coated tablet, trilayer tablet, bilayer tablet, multilayer tablet, orally disintegrating tablet, mini tablet, pellet, sugar pellet, buccal tablet, sublingual tablet, effervescent tablet, immediate release tablet, modified release tablet, film-coated tablet, gastric disintegrating tablet, pill, capsule, oral granule, powder, coated bead system, microsphere, tablet in tablet, inlay tablet, dragee, sachet, orally administrable film.

In a preferred embodiment, the composition is in the form of a film-coated tablet.

According to this embodiment, the film-coating has a thickness of 1 to 120 µm. Preferably the film-coating has a thickness of 50 to 110 µm.

It has been surprisingly found that using the film coating in a specific thickness takes part in enhancing the stability of the composition comprising ivabradine hydrochloride or ivabradine oxalate or pharmaceutically acceptable polymorphs thereof.

Suitable film-coating layer may also preferably be used for the protection from the moisture, light and oxygen. It can be selected from the group comprising polyvinyl alcohol-polyethylene glycol copolymers (Kollicoat IR); polyvinyl alcohol or copolymers or mixtures thereof (Opadry AMB II); polyvinylpyrrolidone-vinyl acetate copolymers (Kollidon VA 64); ethylcellulose dispersions (Surelease); polyethylene glycol; polyvinyl alcohol and lecithin (Sheffcoat PVA+); polyvinylprolidone and all kinds of OpadryTM, as well as pigments, dyes, titanium dioxide, iron oxide, talc or polymethylmetacrylate copolymers (Eudragit).

According to one embodiment, the composition comprises at least one pharmaceutically acceptable excipient selected from fillers, disintegrants, binders lubricants, glidants, melting components or mixtures thereof.

Suitable fillers are selected from microcrystalline cellulose, lactose monohydrate, dibasic calcium phosphate, lactose, mannitol, spray-dried mannitol, starch, dextrose, sucrose, fructose, maltose, sorbitol, xylitol, inositol, kaolin, inorganic salts, polysaccharides, sodium chloride, dextrates, lactitol, maltodextrin, sucrose-maltodextrin mixture, trehalose, sodium carbonate, sodium bicarbonate, calcium carbonate or mixtures thereof.

In a preferred embodiment, the filler is selected from microcrystalline cellulose, lactose monohydrate, dibasic calcium phosphate, lactose or mixtures thereof.

Acoording to this embodiment, microcrystalline cellulose is present in an amount of between 5.0% and 90.0%, preferably between 5.0% and 70.0% by weight of the total formulation, lactose is present in an amount of between 5.0% and 90.0%, preferably between 5.0% and 70.0% by weight of the total formulation, lactose monohydrate is present in an amount of between 5.0% and 90.0%, preferably between 5.0% and 70.0% by weight of the total formulation, dibasic calcium phosphate is present in an amount of between 5.0% and 50.0% by weight of the total formulation.

Particle size distribution of the filler play a significant role for the qualification of the composition subjected to the invention. As used herein, 'particle size distribution' means the cumulative volume size distribution as tested by any conventionally accepted method such as the laser diffraction method (Malvern analysis).

Laser diffraction measures particle size distributions by measuring the angular variation in intensity of light scattered as a laser beam passes through a dispersed particulate sample. Large particles scatter light at small angles relative to the laser beam and small particles scatter light at large angles, as illustrated below. The angular scattering intensity data is then analyzed to calculate the size of the particles responsible for creating the scattering. The particle size is reported as a volume equivalent sphere diameter.

According to this measuring method, the term D10 means, the size at which 10% by volume of the particles are finer and D50 means the size at which 50% by volume of the particles are finer and D90 means the size at which %90 by volume of the particles are finer.
The D50 value of the filler is ranging between 1 to 200 µm. Preferably, this value is ranging between 60 to 190 µm.

The D90 value of the filler is ranging between 1 to 500 µm. Preferably this value is ranging between 100 to 375 µm.

The D10 value of the filler is ranging between 1 to 50 µm. Preferably this value is ranging between 20 to 40 µm.

In a preferred embodiment, the filler is microcrystalline cellulose. The D90 value of microcrystalline cellulose is ranging between 1 to 500 µm. Preferably this value is ranging between 200 to 300 µm. The D50 value of microcrystalline cellulose is ranging between 1 to 200 µm. Preferably this value is ranging between 100 to 200 µm.

In a preferred embodiment, the filler is lactose. The D90 value of lactose is ranging between 1 to 500 µm. Preferably this value is ranging between 300 to 400 µm. The D50 value of lactose is ranging between 1 to 200 µm. Preferably this value is ranging between 150 to 200 µm.

In a preferred embodiment, the filler is dibasic calcium phosphate. The D90 value of dibasic calcium phosphate is ranging between 1 to 300 µm. Preferably this value is ranging between 50 to 110 µm. The D50 value of dibasic calcium phosphate is ranging between 1 to 200 µm. Preferably this value is ranging between 50 to 100 µm.

These specific selections of particle size distribution values, especially the D90 value range of the filler surprisingly ensure the uniformity of the content of active substance of ivabradine-containing pharmaceutical compositions.

Suitable disintegrants are selected from a group comprising cross-linked carboxymethyl cellulose (croscarmellose sodium), sodium starch glycolate, pregelatinized starch, microcrystalline cellulose, crospovidone (cross-linked polyvinil pyrrolidone), povidone, poloxamer, low-substituted hydroxypropyl cellulose, starch, sodium carboxymethyl cellulose, calcium carboxymethyl cellulose, carboxymethyl cellulose, docusate sodium, polyacrylate potassium, sodium alginate, alginic acid, alginates, ion-exchange resins, poloxamer, sodium glycine carbonate, sodium lauryl sulphate or mixtures thereof,

In a preferred embodiment, the disintegrant is selected from cross-linked carboxymethyl cellulose (croscarmellose sodium), sodium starch glycolate, pregelatinized starch or mixtures thereof.

Suitable binders are selected from a group comprising xanthan gum, guar gum, carbomer, pregelatinized starch, povidone, copovidone, carnauba wax, hydroxypropyl methyl cellulose, pullulan, polymethacrylate, glyceryl behenate, hydroxypropyl cellulose, carboxymethyl cellulose, methyl cellulose, hydroxyethyl cellulose, sodium carboxymethyl cellulose, carboxymethyl cellulose calcium, ethyl cellulose, microcrystalline cellulose, polymethacrylates, polyethylene oxide, polyvinyl alcohol, polycarbophil, polyvinyl acetate and its copolymers, gelatin, starch, alginate, carrageen, kollagen, agar, pectin, hyaluronic acid, cellulose acetate phthalate, hydroxypropyl starch, hydroxyethyl methyl cellulose, polaxomer, polyethylene glycol, sugars, glycose syrups, natural gums, tragacanth gum, polyacrylamide, aluminum hydroxide, benthonite, laponite, cetostearyl alcohol, polyoxyethylene-alkyl ethers, acacia mucilage, polydextrose or mixtures thereof.

In a preferred embobiment, the binder is selected from xanthan gum, guar gum, carbomer, or mixtures thereof.

Suitable lubricants are selected from a group comprising magnesium stearate, sodium stearyl fumarate, calcium stearate, zinc stearate, talc, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyceryl palmitostearate, sodium lauryl sulphate or mixtures thereof.

In a preferred embodiment, the lubricant is selected from magnesium stearate, sodium stearyl fumarate or mixtures thereof.

According to this embodiment, magnesium stearate is present in an amount of between 0.1% and 10.0%, preferably between 0.1% and 5.0% by weight of the total formulation, sodium stearyl fumarate is present in an amount of between 0.1% and 10.0% by weight of the total formulation.

Suitable glidants are selected from colloidal silicon dioxide, talc, aluminium silicate or mixtures thereof.

In a preferred embodiment, the glidant is colloidal silicon dioxide or talc.

According to this embodiment, the glidant is present in an amount of between 0.1 and 12.0%, preferably between 0.5 and 12.0% by weight of the total formulation.

Suitable melting components are selected from gelucire (stearyl macrogolglyceride), poloxamer (polyoxyethylene-polyoxypropylene block copolymer), polyethylene glycol, povidone, soluplus, cationic methacrylate, copovidone, methacrylic acid copolymers, cellulose acetate phthalate, acetylated monoglyceride, butil pthalybutyl glycolate, dibutyl tartrate, diethyl phthalate, dimethly phthalate, ethyl phthalylethly glycolate, glycerin, propylene glycol, triacetin, triacetin citrate, tripropionin or mixtures thereof.

In a preferred embodiment, the melting component is gelucire (stearyl macrogolglyceride).

Suitable polymorphs of ivabradine hydrochloride are selected from form S, form delta, form delta D, form alfa, form beta, form beta D, form gamma, form gamma D, form I, form X, form II, form IV, and Epsilon.

Preferably, suitable polymorphs of ivabradine hydrochloride are form S, form delta and form delta D.

**Example 1: Film-coated tablet**

| **Ingredients** | **Amount (%)** |
|---|---|
| Ivabradine hydrochloride or ivabradine oxalate or pharmaceutically acceptable polymorphs thereof | 0.01-20.00 |
| Microcrystalline cellulose (Avicel® PH 112) | 5.00-90.00 |
| Dibasic calcium phosphate | 5.00-50.00 |
| Carbomer | 0.50-10.00 |
| Pregelatinized starch | 1.00-30.00 |
| Colloidal silicon dioxide or talc | 0.10-12.00 |
| Magnesium stearate | 0.10-10.00 |
| **Total tablet** | **100** |
| Opadry AMB II or Kollicoat IR | 1.00-5.00 |
| **Total film**-**coated tablet** | **101.00-105.00** |

The pharmaceutical compositions mentioned above are prepared by following these steps:
a. Mixing ivabradine hydrochloride or ivabradine oxalate or pharmaceutically acceptable polymorphs thereof, dibasic calcium phosphate, microcrystalline cellulose, carbomer and pregelatinized starch.
b. Adding colloidal silicon dioxide or talc to the mixture and mixing until a homogenous mixture is achieved.
c. Adding magnesium stearate to the mixture and mixing for 1-2 minutes.
d. Compressing the powder mixture to form tablets
e. Coating these tablets with Opadry AMB II or Kollicoat IR.

**Example 2: Film-coated tablet**

| **Ingredients** | **Amount (%)** |
|---|---|
| Ivabradine hydrochloride or ivabradine oxalate or pharmaceutically acceptable polymorphs thereof | 0.01-20.00 |
| Microcrystalline cellulose (Avicel® PH 112) | 5.00-90.00 |
| Croscarmellose sodium | 0.50-15.00 |
| Lactose monohydrate | 5.00-90.00 |
| Guar gum | 0.50-10.00 |
| Colloidal silicon dioxide or talc | 0.10-12.00 |
| Magnesium stearate | 0.10-10.00 |
| **Total tablet** | **100** |
| Opadry AMB II or Kollicoat IR | 1.00-5.00 |
| **Total film**-**coated tablet** | **101.00-105.00** |

The pharmaceutical compositions mentioned above are prepared by following these steps:
a. Mixing ivabradine hydrochloride or ivabradine oxalate or pharmaceutically acceptable polymorphs thereof, half of microcrystalline cellulose, lactose monohydrate and half of croscarmellose sodium.
b. Dissolving guar gum in water
c. Granulating the powder mixture with this guar gum solution
d. Drying the granules
f. Mixing the dried granules, the other half of microcrystalline cellulose and the other half of croscarmellose and colloidal silicon dioxide or talc until a homogenous mixture is achieved
e. Adding magnesium stearate to the mixture and mixing for 1-2 minutes
f. Compressing the powder mixture to form tablets
g. Coating these tablets with Opadry AMB II or Kollicoat IR.

**Example 3: Film-coated tablet**

| **Ingredients** | **Amount (%)** |
|---|---|
| Ivabradine hydrochloride or ivabradine oxalate or pharmaceutically acceptable polymorphs thereof | 0.01-20.00 |
| Microcrystalline cellulose (Avicel® PH 112) | 5.00-90.00 |
| Croscarmellose sodium | 0.50-15.00 |
| Dibasic calcium phosphate | 5.00-50.00 |
| Xanthan gum | 0.05-10.00 |
| Colloidal silicon dioxide or talc | 0.10-12.00 |
| Magnesium stearate | 0.10-10.00 |
| **Total tablet** | **100** |
| Opadry AMB II or Kollicoat IR | 1.00-5.00 |
| **Total film**-**coated tablet** | **101.00-105.00** |

The pharmaceutical compositions mentioned above are prepared by following these steps:
a. Mixing ivabradine hydrochloride or ivabradine oxalate or pharmaceutically acceptable polymorphs thereof, dibasic calcium phosphate, microcrystalline cellulose, croscarmellose sodium and colloidal silicon dioxide or talc.
b. Passing the powder mixture from the roller compactor and sieving
c. Adding magnesium stearate to the granules and mixing for 1-2 minutes
d. Compressing the powder mixture to form tablets
e. Coating these tablets with Opadry AMB II or Kollicoat IR.

**Example 4: Film-coated tablet**

| **Ingredients** | **Amount (%)** |
|---|---|
| Ivabradine hydrochloride or ivabradine oxalate or pharmaceutically acceptable polymorphs thereof | 0.01-20.00 |
| Microcrystalline cellulose (Avicel® PH 112) | 5.00-90.00 |
| Sodium starch glycolate | 0.10-15.00 |
| Lactose | 5.00-90.00 |
| Gelucire | 0.50-20.00 |
| Colloidal silicon dioxide or talc | 0.10-12.00 |
| Sodium stearyl fumarate | 0.10-10.00 |
| **Total tablet** | **100** |
| Opadry AMB II or Kollicoat IR | 1.00-5.00 |
| **Total film**-**coated tablet** | **101.00-105.00** |

The pharmaceutical compositions mentioned above are prepared by following these steps:
a. Mixing ivabradine hydrochloride or ivabradine oxalate or pharmaceutically acceptable polymorphs thereof and gelucire
b. Melting the mixture
c. Passing the mixture from the extruder
d. Cooling the mixture and sieving
e. Adding lactose, microcrystalline cellulose, sodium starch glycolate and colloidal silicon dioxide or talc to the granules and mixing
f. Adding sodium stearyl fumarate to the granules and mixing for a short time
g. Compressing the powder mixture to form tablets
h. Coating these tablets with Opadry AMB II or Kollicoat IR.

## Claims

1. A solid oral pharmaceutical composition comprising ivabradine or a pharmaceutically acceptable salt thereof or pharmaceutically acceptable polymorphs thereof, wherein the pharmaceutically acceptable salt is ivabradine hydrochloride or ivabradine oxalate.

2. The solid oral pharmaceutical composition according to claim 1, wherein the composition comprising ivabradine hydrochloride or ivabradine oxalate or pharmaceutically acceptable polymorphs thereof in an amount of 0.01% to 20.00% by weight of the total formulation.

3. The solid oral pharmaceutical composition according to claim 1 or 2, wherein the composition is in the form of coated tablet, trilayer tablet, bilayer tablet, multilayer tablet, orally disintegrating tablet, mini tablet, pellet, sugar pellet, buccal tablet, sublingual tablet, effervescent tablet, immediate release tablet, modified release tablet, film-coated tablet, gastric disintegrating tablet, pill, capsule, oral granule, powder, coated bead system, microsphere, tablet in tablet, inlay tablet, dragee, sachet, orally administrable film.

4. The solid oral pharmaceutical composition according to claim 3, wherein the composition is in the form of a film-coated tablet.

5. The solid oral pharmaceutical composition according to claim 4, wherein the film coating has a thickness of 1 to 120 µm.

6. The solid oral pharmaceutical composition according to claim 5, wherein the film coating selected from the group comprising polyvinyl alcohol-polyethylene glycol copolymers; polyvinyl alcohol or copolymers or mixtures thereof; polyvinylpyrrolidone-vinyl acetate copolymers; ethylcellulose dispersions; polyethylene glycol; polyvinyl alcohol and lecithin; polyvinylprolidone; polymethylmetacrylate copolymers.

7. The solid oral pharmaceutical composition according to claim 1, wherein the composition further comprising at least one pharmaceutically acceptable excipient which is selected from disintegrants, fillers, binders lubricants, glidants, melting components or mixtures thereof.

8. The solid oral pharmaceutical composition according to claim 7, wherein the filler is selected from microcrystalline cellulose, lactose monohydrate, dibasic calcium phosphate, lactose, mannitol, spray-dried mannitol, starch, dextrose, sucrose, fructose, maltose, sorbitol, xylitol, inositol, kaolin, inorganic salts, polysaccharides, sodium chloride, dextrates, lactitol, maltodextrin, sucrose-maltodextrin mixture, trehalose, sodium carbonate, sodium bicarbonate, calcium carbonate or mixtures thereof, preferably the fillers are microcrystalline cellulose, lactose monohydrate, dibasic calcium phosphate, lactose or mixtures thereof.

9. The solid oral pharmaceutical composition according to claim 8, wherein the D50 value of the filler is ranging between 1 to 200 µm, preferably between 60 to 190 µm.

10. The solid oral pharmaceutical composition according to claim 8, wherein the D90 value of the filler is ranging between 1 to 500 µm, preferably between 100 to 375 µm.

11. The pharmaceutical composition according to any of the preceding claims, comprising:
0.01%-20.00% ivabradine hydrochloride or ivabradine oxalate or pharmaceutically acceptable polymorphs thereof
5.00%-90.00% microcrystalline cellulose
0.50%-15.00% croscarmellose sodium
5.00%-90.00% lactose monohydrate
0.50%-10.00% guar gum
0.10%-12.00% colloidal silicon dioxide or talc
0.10%-10.00% magnesium stearate
1.00%-5.00% coating

12. The pharmaceutical composition according to any of the claims 1 to 10, comprising:
0.01%-20.00% ivabradine hydrochloride or ivabradine oxalate or pharmaceutically acceptable polymorphs thereof
5.00%-90.00% microcrystalline cellulose
5.00%-50.00% dibasic calcium phosphate
0.50%-10.00% carbomer
1.00%-30.00% pregelatinized starch
0.10%-12.00% colloidal silicon dioxide or talc
0.10%-10.00% magnesium stearate
1.00%-5.00% coating

13. The pharmaceutical composition according to any of the claims 1 to 10, comprising:
0.01%-20.00% ivabradine hydrochloride or ivabradine oxalate or pharmaceutically acceptable polymorphs thereof
5.00%-90.00% microcrystalline cellulose
0.50%-15.00% croscarmellose sodium
5.00%-50.00% dibasic calcium phosphate
0.05%-10.00% xanthan gum
0.10%-12.00% colloidal silicon dioxide or talc
0.10%-10.00% magnesium stearate
1.00%-5.00% coating

14. The pharmaceutical composition according to any of the claims 1 to 10, comprising:
0.01%-20.00% ivabradine hydrochloride or ivabradine oxalate or pharmaceutically acceptable polymorphs thereof
5.00%-90.00% microcrystalline cellulose
0.10%-15.00% sodium starch glycolate
5.00%-90.00% lactose
5.00%-20.00% stearyl macrogolglyceride
0.10%-12.00% colloidal silicon dioxide or talc
0.10%-10.00% sodium stearyl fumarate
1.00%-5.00% coating

15. The process for preparation of the pharmaceutical composition according to claim 11, wherein the process comprising the following steps:
a. Mixing ivabradine hydrochloride or ivabradine oxalate or pharmaceutically acceptable polymorphs thereof, half of microcrystalline cellulose, lactose monohydrate and half of croscarmellose sodium.
b. Dissolving guar gum in water
c. Granulating the powder mixture with this guar gum solution
d. Drying the granules
f. Mixing the dried granules, the other half of microcrystalline cellulose and the other half of croscarmellose sodium and colloidal silicon dioxide or talc until a homogenous mixture is achieved
e. Adding magnesium stearate to the mixture and mixing for 1-2 minutes
f. Compressing the powder mixture to form tablets
g. Coating these tablets with polyvinyl alcohol or copolymers or polyvinyl alcohol-polyethylene glycol copolymers

16. The process for preparation of the pharmaceutical composition according to claim 12, wherein the process comprising the following steps:
a. Mixing ivabradine hydrochloride or ivabradine oxalate or pharmaceutically acceptable polymorphs thereof, dibasic calcium phosphate, microcrystalline cellulose, carbomer and pregelatinized starch.
b. Adding colloidal silicon dioxide or talc to the mixture and mixing until a homogenous mixture is achieved.
c. Adding magnesium stearate to the mixture and mixing for 1-2 minutes.
d. Compressing the powder mixture to form tablets
e. Coating these tablets with polyvinyl alcohol or copolymers or polyvinyl alcohol-polyethylene glycol copolymers
